# EUROPEAN PATENT APPLICATION

(11) **EP 2 505 194 A2**
(43) Date of publication of application: **03.10.2012**
(21) Application number: 12161080.2
(22) Date of filing: 23.03.2012
(51) Int. Cl.: A61K 9/70, A61K 47/10, A61K 47/14, A61K 47/22, A61K 31/465

(54) **Nicotine-containing patch preparation**

(30) Priority: 01.04.2011 JP 2011082237
(71) Applicant: Nitto Denko Corporation, Osaka 567-8680 (JP)
(72) Inventor: Enomoto, Kazumi, Osaka, Ibaraki 567-8680 (JP); Kuroda, Hidetoshi, Osaka, Ibaraki 567-8680 (JP); Satoda, Shiro, Osaka, Ibaraki 567-8680 (JP); Nakamura, Koji, Osaka, Ibaraki 567-8680 (JP); Takita, Tomohito, Osaka, Ibaraki 567-8680 (JP)
(74) Representative: Duckworth, Timothy John

(57) **Abstract**

An object of the present invention is to provide a nicotine-containing patch preparation which enables to suppress degradation of nicotine and coloring of the patch preparation. The present invention provides a patch preparation comprising a support and an adhesive layer on its at least one surface, wherein the adhesive layer comprises an adhesive polymer, nicotine and a stabilizer, and the stabilizer is at least one kind selected from the group consisting of butylhydroxyanisole (BHA), propyl gallate (PGA), dibutylhydroxytoluene (BHT) and 2-mercaptobenzimidazole (MBT).

## Description

### FIELD OF THE INVENTION

The present invention relates to a patch preparation in which an adhesive layer containing nicotine is provided on at least one side of a support.

### [BACKGROUND OF THE INVENTION]

Conventionally, patch preparations to transdermally administer nicotine are known. Since nicotine is easily oxidized, a nicotine-containing patch preparation may cause some problems such as degradation of nicotine and coloring (yellowing) of the patch preparation depending on formulations.

To cope with these problems, in JP-A-2008-208084, a method including storing a nicotine-containing patch preparation by housing and sealing it in a package filled with nitrogen gas is described. However, this method cannot prevent degradation of nicotine when a pinhole is made in the package.

### [PRIOR ART REFERENCE]

Patent Document 1 JP-A-2008-208084

### [SUMMARY OF THE INVENTION]

The present invention has been made in view of the above situation, and an object thereof is to provide a nicotine-containing patch preparation which can prevent degradation of nicotine, coloring of patch preparation and the like.

### [THE MEANS TO SOLVE THE PROBLEM]

Accordingly, the present invention provides:
1. a patch preparation comprising a support and an adhesive layer which is provided on at least one surface of the support, wherein the adhesive layer comprises an adhesive polymer, nicotine and a stabilizer, and the stabilizer is at least one kind selected from the group consisting of butylhydroxyanisole (BHA), propyl gallate (PGA), dibutylhydroxytoluene (BHT) and 2-mercaptobenzimidazole (MBI);
2. the patch preparation according to the above-mentioned 1, wherein the adhesive polymer comprises an acrylic polymer;
3. the patch preparation according to the above-mentioned 1 or 2, wherein the adhesive layer further comprises a plasticizer;
4. the patch preparation according to any one of the above-mentioned 1- 3, wherein the adhesive layer further comprises a polyol compound;
5. the patch preparation according to any one of the above-mentioned 1 - 4, wherein the adhesive layer is crosslinked; and
6. the patch preparation according to any one of the above-mentioned 1 to 5, which is for storing in an atmosphere with an oxygen concentration of 5-25% by volume.

### [EFFECT OF THE INVENTION]

The present invention enables to suppress degradation of nicotine and generation of nicotine analogue, by including a specific stabilizer into a nicotine-containing patch preparation. Thereby, the nicotine content in the nicotine-containing patch preparation may be maintained, and coloring (yellowing) of the nicotine-containing patch preparation may be suppressed. As used herein, "nicotine analogue" means a substance derived from nicotine.

When the nicotine-containing patch preparation of the present invention is stored by housing and sealing in a package, degradation of nicotine and coloring of the patch preparation may be suppressed even if the package is broken.

### [EMBODIMENT TO CARRY OUT THE INVENTION]

The present invention will be explained referring to preferred embodiments below. However, the explanation to the preferred embodiments is just of illustrative nature, and shall not be construed to limit the present invention, as well as the application and the use thereof.

In the patch preparation of the present invention, an adhesive layer contains nicotine. Nicotine may be in any forms such as a free base or a salt (e.g., a hydrochloride salt). In view of transdermal absorption, nicotine in a free base form (hereinafter occasionally referred to as "nicotine free base") is preferred.

The amount of nicotine may be e.g., 0.1-40% by weight, preferably 0.5-20% by weight, more preferably 1.0-15% by weight, further preferably 5.0-10% by weight based on the total weight of the adhesive layer. When the amount is less than 0.1% by weight, a sufficient amount of nicotine may not be released to afford a sufficient therapeutic effect. When the amount is more than 40% by weight, a therapeutic effect may be limited, and there is a possibility of economically disadvantage.

In the patch preparation of the present invention, the adhesive layer contains a stabilizer. The stabilizer is at least one kind selected from the group consisting of butylhydroxyanisol (BHA), propyl gallate (PGA), dibutylhydroxytoluene (BHT) and 2-melcaptobenzimidazole (MBI). Here, the butylhydroxyanisole (BHA) is a trivial name for a mixture of 2-tert-butyl-4-hydroxyanisole and 3-tert-butyl-4-hydroxyanisole, and the dibutylhydroxytoluene (BHT) is a trivial name for 3,5-di-tert-butyl-4-hydroxytoluene. Of these, butylhydroxyanisole (BHA), propyl gallate (PGA) and dibutylhydroxytoluene (BHT) are preferred. The butylhydroxyanisole (BHA) is more preferred because only a small amount thereof may exhibit a sufficient nicotine stabilizing effect.

The amount of the stabilizer is not particularly limited so far as the amount does not adversely affect a physical property of the adhesive layer. When the amount is too much, the property such as adhesiveness of the adhesive layer may be lowered. When the amount is too small, a sufficient stabilizing effect may not be occurred. Thus, the amount of the stabilizer is preferably 0.001-10% by weight, more preferably 0.005-5.0% by weight, most preferably 0.01-1.05% by weight based on the total weight of the adhesive layer.

In the present invention, the adhesive layer contains an adhesive polymer. Examples of the adhesive polymer include a rubber polymer, an acrylic polymer and the like. In addition, a blend of a tackifying agent (tackifier) and a polymer free of adhesiveness (e.g., rubber polymer) can also be used as an adhesive polymer.

Most of the rubber polymers do not have a highly reactive functional group. Thus, nicotine tends to be relatively stably maintained in the adhesive layer containing a rubber polymer as an adhesive polymer, and a nicotine analogue is less apt to be generated in the adhesive layer. Examples of the rubber polymer include silicone rubber, polyisoprene, polyisobutylene, styrene-butadiene block copolymer, styreneisoprene-styrene block copolymer, styrene-butadiene-styrene block copolymer and the like.

The acrylic polymer has a relatively high degree of freedom in controlling its adhesiveness or drug solubility depending on the kind and ratio of monomers subjected to copolymerization. However, the acrylic polymer may have a functional group which shows reactivity with nicotine in its polymer chain, and a monomer or a polymerization initiator which remains therein may react with nicotine. Thus, in an adhesive layer containing the acrylic polymer as its adhesive polymer, degradation of nicotine or generation of nicotine analogue may increase. In this respect, the present invention may advantageously be effected in an embodiment using the acrylic polymer, since the patch preparation of the present invention may effectively suppress degradation of nicotine and generation of nicotine analogue by the stabilizer.

An acrylic polymer containing (meth)acrylic acid alkyl ester is preferred, and an acrylic polymer containing a (meth)acrylic acid alkyl ester as the main component (main constituting unit) is more preferred. As used herein, the main component means that the amount of the (meth)acrylic acid alkyl ester is not less than 50% by weight in the acrylic polymer. A copolymer of a (meth) acrylic acid alkyl ester as the main component (first monomer component) and a vinyl monomer having a functional group capable of being involved in a crosslinking reaction (second monomer component), and a copolymer of the first monomer component, the second monomer component and the other monomer (third monomer component) is particularly preferred in view of easiness in crosslinking treatment, adhesiveness to human skin and operability (e.g., drug solubility).

Examples of the alkyl group of the (meth) acrylic acid alkyl ester (first monomer component) include a linear, branched chain or cyclic alkyl group having 1 to 18 carbon atoms (e.g., methyl, ethyl, propyl, butyl, pentyl, hexyl, cyclohexyl, heptyl, octyl, 2-ethylhexyl, nonyl, decyl, undecyl, dodecyl, tridecyl and the like). Of these, a linear, branched chain or cyclic alkyl group having 4-18 carbon atoms (e.g., butyl, pentyl, hexyl, cyclohexyl, heptyl, octyl, 2-ethylhexyl, nonyl, decyl, undecyl, dodecyl, tridecyl and the like) is preferred. A monomer component to lower a glass transition temperature of the polymer is preferred to afford the polymer adhesiveness at a room temperature. For this purpose, a linear, branched chain or cyclic alkyl group having 4 to 8 carbon atoms (e.g., butyl, pentyl, hexyl, cyclohexyl, heptyl, octyl, 2-ethylhexyl and the like) is more preferred, butyl, 2-ethylhexyl, cyclohexyl is further preferred, and 2-ethylhexyl is particularly preferred. Specifically, (meth) acrylic acid alkyl ester includes butyl acrylate, 2-ethylhexyl acrylate, 2-ethylhexyl methacrylate, cyclohexyl acrylate, cyclohexyl methacrylate, and 2-ethylhexyl acrylate is more preferred. These (meth) acrylic acid alkyl esters (first monomer component) may be used alone or in combination of two or more kinds thereof.

Examples of the functional group capable of being involved in a crosslinking reaction of a vinyl monomer (second monomer component) include hydroxyl group, carboxyl group, vinyl group and the like, and, of these, hydroxyl group, or carboxyl group is preferred. Examples of the vinyl monomer having a functional group capable of being involved in a crosslinking reaction include hydroxyethyl (meth) acrylate, hydroxypropyl (meth) acrylate, (meth) acrylic acid, itaconic acid, maleic acid, maleic acid anhydride, methaconic acid, citraconic acid, and glutaconic acid and the like. Of these, acrylic acid, methacrylic acid, or hydroxy ethyl acrylic acid ester (e.g., 2-hydroxyethyl acrylate) is preferred, and acrylic acid is more preferred. The vinyl monomer having a functional group capable of being involved in a crosslinking reaction may be used alone or in combination of two or more kinds thereof.

The other monomer (third monomer component) is used to adjust cohesiveness of the adhesive layer and to adjust solubility or release of nicotine. Examples of the other monomer (third monomer component) include vinyl esters such as vinyl acetate, vinyl propionate and the like; vinyl ethers such as methyl vinyl ether, ethyl vinyl ether and the like; vinyl amides such as N-vinyl-2-pyrrolidone, and N-vinyl caprolactam; (meth)acrylic acid alkoxy esters such as methoxyethyl (meth)acrylate, ethoxyethyl (meth)acrylate, tetrahydrofurfuryl (meth)acrylate and the like; amide group-containing (meth)acrylic acid derivatives such as (meth)acrylamide, dimethyl (meth)acrylamide, N-butyl (meth)acrylamide, N-methylol (meth)acrylamide and the like; (meth)acrylic acid aminoalkyl esters such as aminoethyl (meth)acrylate, dimethylaminoethyl (meth)acrylate, t-butylaminoethyl (meth)acrylate and the like; (meth)acrylic acid alkoxyalkyleneglycol esters such as methoxyethyleneglycol (meth)acrylate, methoxydiethyleneglycol (meth)acrylate, methoxypolyethyleneglycol (meth)acrylate, methoxypolypropyleneglycol (meth)acrylate and the like; (meth)acrylonitrile; sulfo group-containing monomer such as styrenesulfonic acid, allylsulfonic acid, sulfopropyl (meth)acrylate, (meth)acryloyloxynaphthalenesulfonic acid, 2-acrylamido-2-methylpropanesulfonic acid and the like; vinyl-group containing monomers such as vinyl piperidone vinyl pyrimidine, vinyl piperazine, vinyl pyrrole, vinyl imidazole, vinyl oxazole vinyl morpholine and the like. Of these, vinyl esters and vinyl amides are preferred. Vinyl acetate is more preferred among vinyl esters, and N-vinyl-2-pyrrolidone is more preferred among vinyl amides. The other monomer (third monomer component) may be used alone or in combination of two or more thereof.

When the acrylic polymer is a copolymer of the (meth)acrylic acid ester alkyl ester (first monomer component) and a vinyl monomer having a functional group capable of being a crosslinking reaction (second monomer component), the weight ratio of the first monomer component to the second monomer component (the first monomer component: the second monomer component) is preferably 99-85:1-15, more preferably 99-90:1-10.

When the acrylic polymer is a copolymer of the (meth)acrylic acid ester alkyl ester (first monomer component), a monomer having a functional group capable of being a crosslinking reaction (second monomer component), and the other monomer (third monomer component), the weight ratio of the first monomer component, the second monomer component and the third monomer component (the first monomer component:the second monomer component:the third monomer component) is preferably 40-94:1-15:5-50, more preferably 50-89:1-10:10-40.

A polymerization reaction is not particularly limited and can be effected according to methods known per se. Examples of the reaction is adding a polymerization initiator (e.g., benzoyl peroxide, azobisisobutyronitrile) to the aforementioned monomers in a solvent (e.g., ethyl acetate) and reacting them at 50-70°C for 5-48 hours.

A preferred acrylic polymer may be e.g., a copolymer of 2-ethylhexyl acrylate/acrylic acid/n-vinyl-2-pyrrolidone, a copolymer of 2-ethylhexyl acrylate/2-hydroxyethyl acrylate/vinyl acetate, and 2-ethylhexylacrylate/acrylic acid, and a copolymer of 2-ethylhexyl acrylate/acrylic acid/n-vinyl-2-pyrrolidone is more preferred.

The average molecular weight of the adhesive polymer is not particularly limited. However, the weight-average molecular weight (Mw) of the adhesive polymer (particularly, the acrylic polymer) is e.g., 500,000-5,000,000, preferably 1,000,000-3,000,000. The weight-average molecular weight may be measured by use of a multi-angle light scattering detector (MALS). Specifically, the weight-average molecular weight may be measured by a MALS used in condition that Wyatt Technology, DAWN DSP is eluent:THF, flow rate:0.6mL/min, lazar wave length:632.8nm and multi-angle fit method:Berry method. Polystyrene (PS, 29,810) is used to measure a normalize factor of MALS.

The concentration of the adhesive polymer is preferably 30-70% by weight, more preferably 40-70% by weight, most preferably 50-60% by weight. When the concentration is lower than 30% by weight, adhesiveness to skin may be decreased. When it is higher than 70% by weight, irritation to skin may be occurred upon peeling off.

A plasticizer may be contained in the adhesive layer of the present invention to give a soft feeling to the adhesive layer to reduce the pain due to the adhesive force on peeling off the adhesive patch from the skin and to reduce skin irritation.

Examples of the plasticizer include medium-chain a triglyceride; vegetable oils such as olive oil, ricinus and the like; squalene, lanolin. The plasticizer may be used alone or in combination of two or more thereof.

A medium-chain aliphatic acid triglyceride is preferred as the plasticizer. The medium-chain aliphatic acid triglyceride may mildly adjust a transdermal absorption rate of nicotine and is suitable for the present invention. The medium-chain aliphatic acid triglyceride as used herein means a triglyceride of a saturated or unsaturated aliphatic acid having 9-12 carbon atoms. Examples of a aliphatic acid component of the medium-chain triglyceride fatty acids include caprylic acid (octanoic acid, C8), pelargonic acid (nonanoic acid, C9), capric acid (decanoic acid, C10), lauric acid (C12) and the like. The aliphatic acid component may be used alone or in combination of two or more thereof.

When the plasticizer is used, the amount is preferably 10-70% by weight, more preferably 20-60% by weight, further preferably 30-50% by weight based on the total weight of the adhesive layer. When the amount is lower than 10% by weight, it might be difficult to reduce a pain and irritation on skin caused by adhesiveness to the skin upon peeling of the patch preparation form the skin. The amount is higher than 70% by weight, it might be necessary to take an initial administration amount of nicotine into consideration to expedite transdermal administration rate of nicotine.

Polyol compound may be contained in the adhesive layer to give soft feeling to the adhesive layer and to stabilize a crosslinking structure of the adhesive layer against lactic acid in sweat. The polyol compound as used herein means a compound having two or more hydroxy group in the molecule.

Examples of polyol compound include diols (glycols) such as ethylene glycol, diethylene glycol, triethylene glycol, dipropylene glycol, 1,3-butanediol, 1,4-butanediol, 2-ethyl-1,3-hexane diol, propylene glycol and the like; triols such as glycerin, 1,2,6-hexane triol and the like; amino alcohols having two or more hydroxyl groups such as diethanolamine, triethanolamine, diisopropanolamine, triisopropanolamine and the like; polyhydric alcohols having two or more hydroxyl groups such as glycerol mono fatty acid ester, sorbitan fatty acid ester, sucrose fatty acid ester and the like. The polyol compound may be used alone or in combination of two or more thereof. The triols such as glycerin and 1,2,6-hexane triol are preferred polyol compounds because they are general as an additive and already used as well as readily available and economically advantageous.

When the polyol compound is used, the amount thereof is preferably 0.01-10% by weight, more preferably 0.05-5.0% by weight, most preferably 0.1-1.0% by weight based on the total weight of the adhesive layer. When the amount is lower than 0.01% by weight, it might be insufficient to stabilize the crosslinking structure of the adhesive layer. The amount is higher than 10% by weight, it might be difficult to give sufficient cohesiveness to the adhesive layer due to insufficient crosslinking reaction of interest or an adverse effect to the release of nicotine might be occurred.

The adhesive layer of the patch preparation of the present invention may be crosslinked. To crosslink the adhesive layer, steps or processes publicly known *per* se and generally used in the art may be used such as a chemically crosslinking treatment (e.g., crosslinking treatment using a crosslinking agent) or a physically crosslinking treatment (e.g., crosslinking treatment by irradiation of electron beam such as gamma ray or irradiation of ultraviolent light).

Depending on the chemically crosslinking treatment or the physically crosslinking treatment, degradation of nicotine or generation of nicotine analogue during production or storage of the patch preparation may be increased. An embodiment in which the adhesive layer is crosslinked is particularly advantageous because the stabilizer may effectively suppress the degradation of nicotine and generation of nicotine analogue in the patch preparation of the present invention. The chemically crosslinking treatment is preferred in view of less adverse effect to nicotine.

Examples of the crosslinking agent used for the chemical crosslinking treatment are not particularly limited so far as formation of the crosslinking structure is not suppressed by nicotine, and include peroxides (e.g., benzoyl peroxide (BPO) and the like), metal oxides (e.g., magnesium metasilicate aluminate and the like), multifunctional isocyanate compounds, organometallic compounds (e.g., alaninate zinc and zirconium, zinc acetate, zinc glycine ammonium compounds, titanium compounds and the like), metal alcoholate compounds (e.g., tetraethyl titanate, tetraisopropyl titanate, aluminum isopropylate, aluminum sec-butyrate and the like), and metal chelate compounds (e.g., dipropoxybis(acetylacetonate)titanium, tetraoctylene glycol titanium, aluminum isopropylate, ethyl acetoacetate aluminum diisopropylate, aluminum tris (ethyl acetoacetate), aluminum tris (acetylacetonate) and the like). Of these, the metal alcoholate compounds and the metal chelate compounds are preferred in view of effective formation of crosslinking structure in the presence of nicotine. The metal chelate compounds are more preferred because an appropriate density of the crosslinking structure may easily be obtained. Among the metal chelate compounds, ethyl acetoacetate aluminum diisopropylate is particularly preferred. The crosslinking agent may be used alone or in combination of two or more thereof.

When a crosslinking agent is used, the amount thereof which may vary depending on the kinds of the crosslinking agent or the adhesive polymer is preferably 0.01-10% by weight, more preferably 0.05-5.0% by weight based on the total weight of the adhesive layer. When the amount is lower than 0.01% by weight, the number of crosslinking points may not afford sufficient cohesiveness to the adhesive layer to lead glue residue caused by cohesive failure or strong skin irritation upon peeling off. When the amount is more than 10% by weight, skin adhesiveness may become insufficient although cohesiveness may be high. Skin irritation may be caused due to a remaining unreacted crosslinking agent.

The chemical crosslinking treatment may be performed by, e.g., adding a crosslinking agent to the adhesive layer, followed by heating (aging) the adhesive layer at a crosslinking reaction temperature or higher. The heating temperature which may be chosen depending on the kind of the crosslinking agent is preferably 60-90°C, more preferably 60-80°C. A time for the heating is preferably 12-96 hours, more preferably 24-72 hours.

The thickness of the adhesive layer of the patch preparation of the present invention is preferably 20-300 µm, more preferably 25-250 µm, most preferably 50-200 µm. When the thickness is smaller than 20 µm, it may be difficult to afford a sufficient, adhesiveness and to contain an effective amount of nicotine. When the thickness is higher than 300 µm, applying an adhesive composition may be difficult.

The support is not particularly limited in the present invention. However, a support that does not allow passage of nicotine in the adhesive layer to reduce its amount is preferred (i.e., a support impermeable to nicotine). As will be mentioned below, an embodiment in which the adhesive layer contains an organic liquid component, and nicotine and the organic liquid component will not pass through a support to reduce the ratio thereof is preferred (i.e., a support impermeable to the organic liquid component and nicotine).

Examples of the support impermeable to nicotine or the organic liquid component include a film of polyesters (e.g., polyethylene terephthalate (PET) and the like), nylon, polyvinyl chloride, polyethylene, polypropylene, ethylenevinyl acetate copolymers, polytetrafluoroethylene and ionomer resins; and a metal foil and the like. The support may be either a monolayer or a multilayer laminate. A combination of a non-porous layer impermeable to nicotine and consisting of a film or metal foil (preferably a film), and a porous layer capable of improving adhesion to the adhesive layer (anchoring property) is preferable as a laminate.

The porous layer is not particularly limited if the anchoring property with the adhesive layer is improved. Examples of the porous layer include a paper, a woven fabric, a nonwoven fabric (e.g., polyester (e.g., PET) nonwoven fabric) and the like. In addition, non-porous film (e.g., a monolayer or multilayer film of polyester, nylon, saran (trade name), polyethylene, polypropylene, ethylene - vinyl acetate copolymer, polyvinyl chloride, ethylene-ethyl acrylate copolymer, polytetrafluoroethylene, polyethylene terephthalate and the like); nonporous metal foil; as well as those that have been mechanically perforated laminate thereof can be used as a porous layer. Among these, in view of flexibility, a paper, a woven fabric, nonwoven fabric (e.g., polyester (PET for example) nonwoven fabric)) is preferred.

The thickness of the whole support is not particularly limited but preferably 2-500 µm, more preferably 10-50 µm. When the thickness is less than 2 µm, the handling property such as self-supporting property may become worse. When the thickness is more than 500 µm, the compliance may become worse to cause skin discomfort when the patch preparation is adhered to the skin. When the support is a laminate, the thickness of the porous layer is e.g., 10-200 µm in view of improving the anchoring property and flexibility of the support. In the case of a thin-type patch preparations such as a plaster type or a adhesive tape type, the thickness of the porous layer is, e.g., 1-200 µm. When a woven fabric or nonwoven fabric is used as a porous layer, the basis weight thereof is each preferably 5-30 g/m² in view of improving the anchoring property. When the support is a laminate, the thickness of the nonporous layer is, e.g., 0.5-6 µm.

In the patch preparation of the present invention, the adhesive surface (the face opposite to the face laminated to the support) is preferably protected through affixed by a release liner immediately before use. The release liner is not particularly limited in the present invention and any release liners publicly know may be used. Examples of the release liner include, e.g., (i) a release liner having a release layer made of a release agent on the surface of a substrate; (ii) a film made of a highly releasing plastic; and (iii) a release liner having a release layer made of a highly releasing plastic on the surface of a substrate. The release layer may be formed on one face or both faces of the substrate.

Examples of the release agent to form the molding layer include but not limited to, e.g., long chain alkyl group-containing polymers, silicone polymers (silicone release agent), fluorinated polymers (fluorine release agent).

Examples of the highly releasing plastic to form the film or the release layer include, e.g., ethylene-α-olefin copolymers (random copolymers or block copolymers) such as polyethylene (e.g., low density polyethylene, linear low density polyethylene), polypropylene, ethylene-propylene copolymers; polyolefin resins of a mixture thereof; Teflon (registered trade mark) and the like.

Examples of the substrate of the release liner having a release layer include, e.g., plastic films such as a polyethylene terephthalate (PET) film, a polyimide film, a polypropylene film, a polyethylene film, a polycarbonate film, a polyester (excluding PET) film and the like; a metallized plastic film in which a metal is deposited on the above-mentioned plastic film; papers such as Japanese paper, western paper, craft paper, glassine paper, fine paper; fibrous materials such as nonwoven fabric, and cloth; and a metal foil.

The thickness of the release liner is not particularly limited but is normally 200 µm or less, preferably 25-100 µm.

The patch preparation of the present invention is manufactured by, e.g., (i) preparing an adhesive composition for application at least containing an adhesive polymer and a stabilizer; (ii) forming an adhesive layer from the adhesive composition for application; (iii) applying nicotine onto the adhesive surface of the adhesive layer, allowing nicotine to impregnate into the adhesive layer to form an adhesive layer containing nicotine; and (iv) laminating the adhesive layer to at least one surface of a support. One embodiment of the manufacturing the patch preparation of the present invention will be explained as follows.

First, the adhesive polymer and the stabilizer are dissolved or dispersed in a solvent to prepare the adhesive composition for application (a composition to form the adhesive layer). A crosslinking agent may be contained in the adhesive composition for application. The adhesive layer may be formed by applying the adhesive composition for application on the support and drying. Alternatively, the adhesive layer may be formed by applying the adhesive composition for application on a release liner and drying. Examples of the solvent for the adhesive composition for application include, e.g., ethyl acetate, toluene, hexane, 2-propanol, methanol, ethanol and water.

When the adhesive layer is chemically crosslinked, that is, when the adhesive layer is crosslinked by a crosslinking agent, heating (aging) is preferably effected to promote the crosslinking of the formed adhesive layer. This temperature for heating is preferably 60-90°C, more preferably 60-80°C.
The time for heating is preferably 12-96 hours, more preferably 24-74 hours.

The patch preparation of the present invention (that is, a laminate of support/nicotine-containing adhesive layer) may be manufactured through applying and impregnating nicotine onto the thus-obtained adhesive layer on the support. Then, the patch preparation in the form of a laminate of support/nicotine-containing adhesive layer/release liner may be manufactured through affixing a release liner on the adhesive surface of this patch preparation. Alternatively, the patch preparation in the form of the laminate of support/nicotine-containing adhesive layer/release liner may be manufactured through applying and impregnating nicotine onto the thus-obtained adhesive layer on the release liner, and then laminating a support onto the adhesive layer.

Examples of the shape of the patch preparation of the present invention is not particularly limited but include, e.g., tape-shaped, sheet-shaped, matrix-type, reservoir-type, membrane-controlled release type and the like. Of these, the tape shaped patch preparation and the sheet-shaped patch preparation tend to be affected by oxygen. In this respect, the embodiment of the tape shaped or sheet shaped patch preparations of the present invention may advantageously be carried out in that degradation of nicotine and generation of nicotine analogue may effectively be suppressed by the stabilizer.

A dose of the patch preparation may vary depending on the kind or the amount of the adhesive in use as well as age, body weight or condition of a patient. For example, the patch preparation containing 2-150 mg of nicotine is adhered to an adult on about 5-120 cm² of skin for about 1-7 days for administration.

The patch preparation of the present invention may be stored under an oxygen-containing atmosphere because nicotine is fully stabilized by the aforementioned stabilizer. For example, the patch preparation of the present invention may be stored under an atmosphere containing 5-25% by volume of oxygen.

The patch preparation of the present invention may be housed and sealed in a package for storage. Degradation of nicotine and generation of nicotine analogue may effectively be suppressed even if a pinhole or a seal failure occurred on the package and air is entered into the package because the patch preparation of the present invention contains the aforementioned stabilizer. In addition, when the patch preparation of the present invention is housed and sealed in the package, it is unnecessary to replace air in the package by an inactive gas such as nitrogen and/or to treat such as deaeration.

The package is preferably made of a laminate having a gas-impermeable film such as a polyester film, a polyacrylonitrile resin film, or a metal foil. The thickness of the film used for the package is conventionally 10-200 µm.

The shape of the package is not particularly limited so far as it may house and seal the patch preparation of the present invention. For example, examples of the package include a laminate of sheets or films sealed in their peripheral areas by heat sealing or an adhesive and a pouch shaped into a baglike shape.

### [Examples]

The present invention will be explained in more detail by specific examples and the present invention shall not be limited thereto. The words "%" and "part" hereafter shall mean "% by weight" and "part by weight", respectively.

### [Evaluation of patch preparation]

Patch preparations obtained by the examples and comparative examples were evaluated as follows.

### Ratio of nicotine after storage

Patch preparations before storage and after storage under the condition described below were extracted by methanol. The extract was analyzed by HPLC following to the condition below.
- HPLC-column: Inertsil (registered trade mark) ODS-3V (4.6 mm I.D. x 15 cm, 5 µm), manufactured by GL Science
- Column temperature: 40°C
- Mobile phase: phosphate buffer/methanol=7/3 (volume/volume)
- Analyzing time: 12 minutes
- Flow rate: about 2.0 mL/minute
- Detection: UV (260 nm)
- Retention time: 10 minutes

The ratio of nicotine after storage was calculated by the following formula from the nicotine weight calculated based on the HPLC peak area.
The ratio of nicotine after storage (%)=100x(nicotine weight of the patch preparation after storage)/(nicotine weight of the patch preparation before storage)

(2) Ratio of impurity in patch preparation before storage The patch preparation before storage and the patch preparation after storage under the condition described below were extracted by methanol. The extract was analyzed by HPLC following to the condition below.
   - HPLC-column: Inertsil (registered trade mark) ODS-3 (4.6 mm I.D. x 15 cm, 3 µm), manufactured by GL Science
   - Column temperature: 45°C
   - Mobile phase: concentration gradient control was conducted by changing the mixing proportion of mobile phase A (phosphate buffer/methanol=9/1 (v/v)) and mobile phase B (phosphate buffer /methanol=3/7 (v/v)) as shown in Table 1 below (after 50 minutes was post-run.).
   - Flow rate: 1.0 mL/minute
   - Detection: UV (260 nm)
   - Retention time
      Nicotinic acid: 2.1 minutes
      Cotinine: 10.8 minutes
      Myosmine: 17.7 minutes
      Nicotine: 21.1 minutes
      β -Nicotyrine: 24.6 minutes
      Impurity A: 29.9 minutes

**[Table 1]**

| Analyzing time(min) | .Mobile phase A (v/v%) | Mobile phase B (v/v%) |
|---|---|---|
| 0-10 | 100 | 0 |
| 10-45 | 100→0 | 0→100 |
| 45-50 | 0 | 100 |
| 50-52 | 0→100 | 100→0 |
| 52-60 | 100 | 0 |

Mobile phase A: phosphate buffer/methanol=9/1 (v/v)
Mobile phase B: phosphate buffer/methanol=3/7 (v/v)

The ratio of impurity after storage (nicotinic acid, cotinine, Myosmine, β-Nicotyrine and impurity A) was calculated by the following formula based on HPLC peak area.
The ratio of impurity after storage (%) = 100x(HPLC peak area of impurity in the preparation after storage)/(HPLC peak area of nicotine in the patch preparation after storage)

### (3) Coloring of patch preparation

Color change of the patch preparation before storage and the patch preparation after storage under the condition as mentioned below was measured by color chromaticity coordinates (L *, a *, b *) on CIE1976L * a * b * color system (L-star, A-star, B-star color system, JIS Z 8729:2004). The Table below shows b * value and ratio (%) calculated by the following formula. The b * value is a yellowness index.
The ratio of b * values of the patch preparation after storage (%) = 100x(b * value of the patch preparation after storage)/(b * value of the patch preparation before storage)

### [Preparation of adhesive polymer]

In an inert gas atmosphere, 75 parts of 2-ethylhexyl acrylate, 3 parts of acrylic acid, 22 parts of N-vinyl-2-pyrrolidone and 0.2 part of azobisisobutyronitrile were solution polymerized in ethyl acetate at 60°C to afford a solution of an acrylic polymer in ethyl acetate (polymer solids: 28%). Hereafter, this will be abbreviated as "the adhesive polymer solution".

### [Component of adhesive layer]

Using the following ingredients, the adhesive layer was formed.
(1) Nicotine
   Nicotine free base
(2) Adhesive polymer
   The acrylic polymer prepared as described above (copolymer of 2-ethylhexyl acrylate (first monomer component), acrylic acid (second monomer component) and N-vinyl-2-pyrrolidone (third monomer component); first monomer component : second monomer component : third monomer component=75:3:22; the weight average molecular weight (Mw): 2 × 10⁶)
(3) Plasticizer
   Triglyceride of capric acid and/or caprylic acid with glycerin
(4) Polyol compound: glycerol
(5) Crosslinking agent: ethyl acetoacetate aluminum diisopropylate
(6) Stabilizer
   Butylhydroxyanisole (BHA) (a mixture of 2-tert-butyl-4-hydroxyanisole and 3-tert-butyl-4-hydroxyanisole)
   Propyl gallate (PGA)
   Dibutylhydroxytoluene (BHT) (3,5-di-tert-butyl-4-hydroxytoluene)
   2-mercaptobenzimidazole (MBI)
   Acetic acid (±) α-tocopherol (α-TL)

### [Examples 1-4 and comparative examples 1 and 2]

The aforementioned adhesive polymer solution, the plasticizer, the polyol compound, the crosslinking agent and the stabilizer were mixed (however, the stabilizer was not mixed in comparative example 1.) to afford an adhesive composition for application. A polyester film one of which surface was peel-treated was used as each release liner. The adhesive composition for application was applied on the peel-treated surface of the release liner such that the thickness of each adhesive layer after drying is 90 µm and was dried for 3 minutes at 100°C to form an adhesive layer.

A laminate obtained by extrusion of a polyester nonwoven fabric (12 g/m² of basis weight) and a polyethylene terephthalate film (2 µm of thickness) were used as each support. The nonwoven fabric surface of the support was adhered to the adhesive surface of the aforementioned adhesive layer. The adhesive layer was crosslinked by keeping it for 48 hours at 60°C to afford a placebo patch preparation which does not contain nicotine.

Then, the release liner of the placebo patch preparation was peeled off to expose the adhesive surface. This was placed on a die head (30 µm of slit width, 65 small of die head) of a desktop die coater and nicotine was directly and uniformly applied on the adhesive surface.

After impregnation of nicotine to the adhesive layer, the adhesive surface was covered by another release liner of a polyester film to afford a nicotine-containing patch preparation with the release liner. Each ratio and kind of the stabilizer of thus-obtained nicotine-containing patch preparation was shown in Table 2 below.

The thus-obtained nicotine-containing patch preparation was housed and sealed in a package made of a laminate of a polyethylene terephthalate film (12 µm of thickness)/an aluminum foil (12 µm of thickness)/a poly acrylonitrile resin film (30 µm of thickness). The atmosphere in the package is air (21 % by volume of oxygen concentration). Each nicotine-containing patch reparation housed and sealed in the package was stored for 2 months at 50°C and then concentration of nicotine after storage, concentration of impurities after storage, b * value and the ratio thereof were measured. The result was shown in Table 3 below.

**[Table 2]**

| | Nicotine | Adhesive polymer | Plasticizer | Polyol compound | Crosslinking agent | Stabilizer | |
|---|---|---|---|---|---|---|---|
| | Amount (part) | Amount (part) | Amount (part) | Amount (part) | Amount (part) | Name | Amount (part) |
| Ex. 1 | 8.86 | 53.99 | 36.5 | 0.46 | 0.09 | BHA | 0.10 |
| Ex. 2 | 8.86 | 53.89 | 36.5 | 0.46 | 0.09 | PGA | 0.20 |
| Ex. 3 | 8.86 | 53.59 | 36.5 | 0.46 | 0.09 | BHT | 0.50 |
| Ex. 4 | 8.86 | 53.47 | 36.5 | 0.46 | 0.09 | MBI | 0.62 |
| Com. Ex. 1 | 8.86 | 54.09 | 36.5 | 0.46 | 0.09 | - | - |
| Com. Ex. 2 | 8.86 | 53.99 | 36.5 | 0.46 | 0.09 | α-TL | 0.10 |

**[Table 3]**

| | Stabilizer | Nicotine ratio after storage (%) | Nicotine impurity ratio after storage (%) | | | | | b* value after storage [Ratio] |
|---|---|---|---|---|---|---|---|---|
| | | | Nicotinic acid | Cotinine | Myosmine | β-Nicotyrine | Impurity A | |
| Ex. 1 | BHA | 100 | 0.2 | 0.2 | 0.4 | 0.2 | N.D. | 10.38 [133%] |
| Ex. 2 | PGA | 98 | 0.0 | 0.1 | 0.2 | 0.0 | N.D. | 8.9 [247%] |
| Ex. 3 | BHT | 95 | 0.1 | 0.2 | 0.3 | 0.1 | N.D. | 8.46 [121%] |
| Ex. 4 | MBI | 99 | N.D. | 0.1 | 0.2 | 0.0 | 1.0 | 5.1 [164%] |
| Comp. Ex. 1 | - | 9 | 2.7 | 1.2 | 4.9 | 8.1 | N.D. | 33.31 [375%] |
| Comp. Ex. 2 | α-TL | 70 | 2.8 | 1.3 | 5.6 | 7.7 | N.D. | 32.84 [356%] |

| | | | | | | | | |
|---|---|---|---|---|---|---|---|---|
| (Remarks) (1) Stored under an atmosphere of 21% by volume of oxygen concentration (2) N.D.: Not detected (3) Impurity A: An impurity derived from MBI | | | | | | | | |

As shown in Table 3, the nicotine ratios after storage of the patch preparations of examples 1-4 which contained butylhydroxyanisole (BHA), propyl gallate (PGA), dibutylhydroxytoluene (BHT) or 2-mercaptobenzimidazole (MBI), respectively, as stabilizers were higher and impurity ratios thereof were lower than those of comparative example 1 which contained no stabilizer and comparative example 2 which contained acetic acid (±) α-tocopherol (α-TL). The coloration (b * values and ratios thereof) of the patch preparations of examples 1-4 were lower than the patch preparations of comparative examples 1 and 2.

1% of ratio of impurity A derived from 2-mercaptobenzimidazole (MBI) was observed in the patch preparation of example 4. To the contrary, impurity A was not observed in the patch preparations 1-3 which contained no MBI. The patch preparation of example 1 which contained butylhydroxyanisole (BHA) as the stabilizer exhibited superior nicotine stabilization effect because its nicotine ratio after storage was the highest despite the smallest amount of the stabilizer.

### [Example 5 and comparative example 3]

Similar to example 1 above, a nicotine-containing patch preparation with a release liner was prepared. Proportion of each component of thus-obtained nicotine-containing patch preparations and stabilizer used therein were shown in Table 4 below.

Each nicotine-containing patch preparation was stored in a similar way to example 1 except that the oxygen concentration was not 21% by volume but 3% by volume. Nicotine ratio, impurity ratio, b * value and its ratio after storage were measured. The results were shown in Table 5.

**[Table 4]**

| | Nicotine | Adhesive polymer | Plasticizer | Polyol compound | Crosslinking agent | Stabilizer | |
|---|---|---|---|---|---|---|---|
| | Amount (parts) | Amount (parts) | Amount (parts) | Amount (parts) | Amount (parts) | Name | Amount (parts) |
| Ex. 5 | 8.86 | 53.59 | 36.5 | 0.46 | 0.09 | BHA | 0.50 |
| Comp. Ex. 3 | 8.86 | 54.09 | 36.5 | 0.46 | 0.09 | - | - |

**[Table 5]**

| | Stabilizer | Nicotine ratio after storage (%) | Nicotine impurity ratio after storage (%) | | | | | b* value after storage [Ratio] |
|---|---|---|---|---|---|---|---|---|
| | | | Nicotinic acid | Cotinine: | Myosmine | β-Nicotyrine | Impurity A | |
| Ex. 5 | BHA | 100 | 0.0 | 0.1 | 0.2 | 0.0 | N.D. | 6.5 [197%] |
| Comp. Ex. 3 | — | 9 | 0.0 | 0.1 | 0.2 | 0.1 | N.D. | 8.5 [258%] |

| | | | | | | | | |
|---|---|---|---|---|---|---|---|---|
| (Remarks) (1) Stored under an atmosphere of 3% by volume of oxygen concentration (2) N.D.: Not detected | | | | | | | | |

As shown in Table 5, the b* value after storage and the ratio thereof of the patch preparation of example 5 which contained butylhydroxyanisole (BHA) as its stabilizer were lower than those of comparative example 3 which contained no stabilizer. These results indicated that the specific stabilizer exhibited nicotine stabilization effect even though the patch preparation was stored under a low oxygen atmosphere (3% by volume of oxygen concentration). However, taking the comparison of example 1 with comparative 1 and the comparison of example 5 with comparative example 3 into consideration, the specific stabilizers more significantly exhibited nicotine stabilization effect if the patch preparations were stored in an atmosphere of a high oxygen concentration (21% by volume of oxygen concentration).

The above explanation was only of an illustrative nature and any modifications of the present invention without apart from the gist of the present invention shall be meant within the scope of the present invention. Any modifications shall not be construed as apart from the spirit and the scope of the present invention.

### [INDUSTRIAL APPLICABILITY]

According to the present invention, a nicotine-containing patch preparation in which nicotine degeneration, generation of nicotine analogue and coloration are suppressed may be obtained. The patch preparation of the present invention may be useful in smoking cessation program for those who wish smoking cessation by transdermal and non-oral administration.

## Claims

1. A patch preparation comprising a support and an adhesive layer which is provided on at least one surface of the support, wherein the adhesive layer comprises an adhesive polymer, nicotine and a stabilizer, and the stabilizer is at least one kind selected from the group consisting of butylhydroxyanisole (BHA), propyl gallate (PGA), dibutylhydroxytoluene (BHT) and 2-mercaptobenzimidazole (MBI).

2. The patch preparation according to claim 1, wherein the adhesive polymer comprises an acrylic polymer.

3. The patch preparation according to claim 1 or 2, wherein the adhesive layer further comprises a plasticizer.

4. The patch preparation according to any one of claims 1 to 3, wherein the adhesive layer further comprises a polyol compound.

5. The patch preparation according to any one of claims 1 to 4, wherein the adhesive layer is crosslinked.

6. The patch preparation according to any one of claims 1 to 5, which is for storing in an atmosphere of 5-25% by volume of oxygen concentration.

7. A method of storing a patch preparation according to any one of claims 1 to 5, comprising storing in an atmosphere of 5-25% by volume of oxygen.

8. Use of a stabilizer selected from BHA, PGA, BHT and MBI to provide storage stability in an atmosphere of 5-25% by volume of oxygen to a patch preparation as defined in any one of claims 1 to 5.
